# EUROPÄISCHE PATENTSCHRIFT

(11) **EP 1 718 222 B1**
(45) Veröffentlichungstag und Bekanntmachung des Hinweises auf die Patenterteilung: **18.11.2009**
(21) Anmeldenummer: 04713134.7
(22) Anmeldetag: 20.02.2004
(51) Int. Cl.: A61B 17/17

(54) **ZIELGERÄT ZUM EINBRINGEN VON WINKELSTABILEN, LANGEN SCHRAUBEN IM ARTIKULÄREN BEREICH EINES KNOCHENS**
AIMING DEVICE FOR INSERTING STABLE-ANGLE, LONG SCREWS IN THE ARTICULAR REGION OF A BONE
APPAREIL DE VISEE DESTINE A L'INTRODUCTION DE VIS LONGUES, ANGULAIREMENT STABLES, DANS LA ZONE ARTICULAIRE D'UN OS

(43) Veröffentlichungstag der Anmeldung: 08.11.2006
(73) Patentinhaber: Synthes GmbH, 4436 Oberdorf (CH)
(72) Erfinder: KÜENZI, Thomas, CH-4127 Birsfelden (CH); ANDERMATT, Daniel, CH-4313 Möhlin (CH); FEIGENWINTER, Gregor, CH-4432 Lampenberg (CH)
(74) Vertreter: Rosenich, Paul
(86) Internationale Anmeldenummer: PCT/IB2004/000441
(87) Internationale Veröffentlichungsnummer: WO 2005/089660

(56) Entgegenhaltungen:
- EP-A- 0 132 284
- EP-A- 1 354 562
- US-A- 4 159 716
- US-A- 4 710 075
- US-A- 5 409 493
- US-A1- 2003 009 171

## Beschreibung

Die Erfindung betrifft ein Zielgerät zum Einbringen von winkelstabilen, langen Schrauben im artikulären Bereich eines Knochens zur optimalen Versorgung von Gelenksfrakturen mit Platten/Schrauben Systemen, am Beispiel eines distalen Humerus.

Zum Stand der Technik sind beispielsweise die Patentschriften US-20020032465-A1, die US-20030220651-A1, die US-4625718, die US-4848327 sowie die WO-03041595-A1 zu erwähnen. Der Oberbegriff vom Anspruch 1 basiert auf dem Inhalt der EP 0 132 284-A1.

Es wird besonders auf die US 2003/0009171 A1 eingegangen. Diese offenbart eine Mehrzahl von Hilfsgeräten zum Einpassen einer Ellenbogenprothese, insbesondere ein Zielgerät zur Bohrung des artikulären Bereichs des Knochens entlang seiner Gelenksachse. Dieses Zielgerät besteht aus einem Bügel, welcher mittels einer Schraubspindel auf dem artikulären Bereich festgeklemmt wird. Eine Führungszone zum Einführen eines Bohrers in den artikulären Bereich entlang der besagten Gelenksachse ist in der Schraubspindel integriert. Die derart herstellbare Bohrung dient hier zum Einbringen eines Stiftes, welcher später die Ausrichtung der Ellenbogenprothese ermöglicht und danach wieder entfernt wird. Das Einbringen einer Schraube ist bei dieser Vorrichtung nicht vorgesehen.

Auch wenn dieses in der US 2003/0009171 A1 geoffenbarte Gerät zu einem komplett anderen Zweck vorgesehen ist, könnte die vom Erfinder beabsichtigte Erstellung von Bohrungen damit unter Umständen durchgeführt werden, wenn auch mit erheblichen Nachteilen verbunden. Das bekannte Gerät bietet keine Möglichkeiten, das Risiko einer Penetration ins Gelenk zu verhindern, was im Zuge der Einpassung einer Ellenbogenprothese auch unerheblich ist, da das Gelenk ohne dies entfernt und ersetzt wird. Bei der Osteosynthese ist jedoch die möglichst schonende Behandlung des Knochens Voraussetzung. Wie schon erwähnt, ist die Setzung der bei der Osteosynthese benötigten distalen Schraube nach vollzogener Bohrung nur möglich, wenn das Gerät oder mindestens die Schraubspindel entfernt würde. Dadurch entfällt jedoch die durch das Gerät bewirkte Kompression zur Gewährleistung des Zusammenhalts allfälliger Knochenfragmente. Während dem gesamten Operationsverlauf mit diesem bekannten Gerät ist somit keine durchgehende Stabilität möglich. Letztlich müsste der Bohrer nachteiligerweise wenigstens die Länge der Schraubspindel und des gewünschten Bohrloches aufweisen, wodurch erhöhte Vibrationen die Bohrung unvorteilhaft beeinflussen können.

Es ist die Aufgabe der vorliegenden Erfindung diese Nachteile zu beheben.

Es soll durch die Erfindung möglich sein, möglichst lange distale Schrauben durch den Knochen im gelenksnahen Bereich des artikulären Blockes gezielt einzubringen, wofür die Bohrungen dementsprechend gezielt und ohne jegliche Penetration ins Gelenk eingebracht werden sollen, wodurch nach Einbringung einer Schraube eine optimale Fixation im guten Knochen erreicht werden soll. Allenfalls gegenüberliegende Schrauben sollen ausserdem gezielt und etwa entlang einer Achse möglichst nahe aneinander vorbeigeführt werden können, ohne eine Kollision zu bewirken. Dies soll insbesondere direkt beim ersten Versuch gewährleistet sein um den Knochen möglichst zu schonen. Ebenfalls ist die Kompression der Knochenfragmente des artikulären Blocks während der gesamten Operationsdauer aufrecht zu erhalten um dadurch für eine stabile Verbindung und gute klinische Resultate zu sorgen.

Das erfindungsgemässe Set gemäß Anspruch 1 besteht unter anderem aus einem Bügel, gegebenenfalls mit diversen Durchbrechungen zur Gewichtseinsparung, und löst die gestellten Aufgaben. Eine bevorzugte Ausführungsform ist wie folgt aufgebaut: Am Bügel ist eine mittels einer Mutter betätigbare Schraubspindel zur Fixierung des Zielgeräts auf dem artikulären Bereich des Knochens vorgesehen. Am gegenüberliegenden Ende des Bügels ist eine Zylinderführung angebracht, welche ihrerseits eine Adaptionsbüchse rotationsbeweglich lagert. Zylinderführung und Adaptionsbüchse sind geeignet, einerseits eine Knochenschraube zu führen, beziehungsweise andererseits eine Bohrbüchse mit vorzugsweise einem Außengewinde, zu lagern. Eine Zielplatte zur Verbindung mit einer Knochenplatte ist dafür vorgesehen, mit der Adaptionsbüchse des Zielgeräts vorübergehend eine Steckverbindung einzugehen.

### Operationsverlauf

Vor der Operation wird die Zielplatte mit einem lateralen Implantat (Knochenplatte) verschraubt. Mittels der Steckverbindung wird die Zielplatte mit dem lateralen Implantat auf der Adaptionsbüchse des Zielgeräts montiert. Die Bohrbüchse wird dann in die Öffnung in der Zylinderführung eingeführt und trifft, durch die Adaptionsbüchse und die Zielplatte hindurch, auf ein komplementäres Innengewinde in einer Bohrung des Implantats. Alles zusammen wird nun auf dem fragmentierten Knochen platziert, durch die Schraubspindel des Zielgeräts festgeklemmt und mit einer proximalen Knochenschraube durch das Implantat hindurch fixiert. Mit Hilfe des erfindungsgemässen Zielgerätes kann somit der Austrittspunkt der distalen, winkelstabilen Schrauben vor dem Bohren bestimmt werden. Wenn alles korrekt ausgerichtet ist, kann durch die integrierte Bohrbüchse hindurch gebohrt werden. Des weiteren kann mit dem Zielgerät, insbesondere anhand der auf dessen Schraubspindel angebrachten Skala direkt die Länge der zu verwendenden Schraube und somit die Tiefe des zu bohrenden Loches bestimmt werden.

Nach dem Entfernen der mit dem Implantat verschraubten Bohrbüchse bleibt die Kompression noch immer über die Steckverbindung zwischen Adaptionsbüchse und Zielplatte bestehen. Dies ist ein wichtiger Vorteil im Unterschied zum Bekannten. Durch Verwendung der Zielplatte ist es also möglich, nach Entfernen der Bohrbüchse, die Schraube bei montiertem Zielbügel, das heisst unter Kompression, einzudrehen. Die entfernte Bohrbüchse gibt jetzt auch den von der Knochenschraube benötigten grösseren Durchmesser frei. Die Knochenschraube, setzt sich mit Ihrem Kopf an dem selben Gewinde des Implantats fest, das zuvor die Bohrbüchse aufgenommen hat. Zudem besteht die Vereinfachung der Ausrichtung von weiteren Schrauben indem durch das Zielgerät die Position der bereits eingebrachten Schraube angezeigt wird, sodass sie ganz gezielt parallel zur Gelenksachse und durch den besten Knochen des distalen Teils eingebracht werden. Wenn dies vollzogen ist, kann das Zielgerät, sowie die Zielplatte entfernt werden.

Weitere Ausbildungen der Erfindung sind in den Figuren und in den abhängigen Patentansprüchen angegeben.

### Die Bezugszeichenliste ist Bestandteil der Offenbarung.

Anhand von Figuren wird die Erfindung symbolisch und beispielhaft näher erläutert.

Die Figuren werden zusammenhängend und übergreifend beschrieben. Gleiche Bezugszeichen bedeuten gleiche Bauteile, Bezugszeichen mit unterschiedlichen Indices geben funktionsgleiche oder ähnliche Bauteile an.

Es zeigen dabei
- Fig.1: -: ein erfindungsgemäßes Set mit separater Bohrbüchse und Zielplatte, in Frontalansicht,
- Fig.2: -: das Set gemäß Fig. 1 mit montierter Bohrbüchse und Zielplatte, in Frontalansicht,
- Fig.3 bis 5:-: die Zielplatte gemäß Fig.1 und 2 allein, in drei unterschiedlichen Ansichten,
- Fig.6: -: eine schematische Darstellung des Zielgerätes mit montierter Bohrbüchse, Zielplatte und damit verschraubtem Implantat, wie es auf dem Humerus angebracht wird in Perspektive, und
- Fig.7: -: eine schematische Darstellung des Humerus mit Implantaten und Knochenschrauben in Frontalebene.

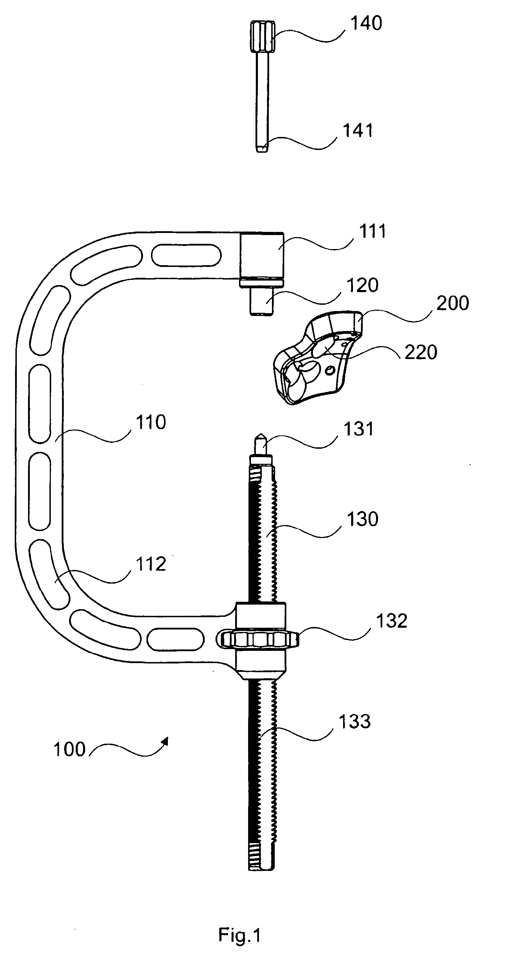

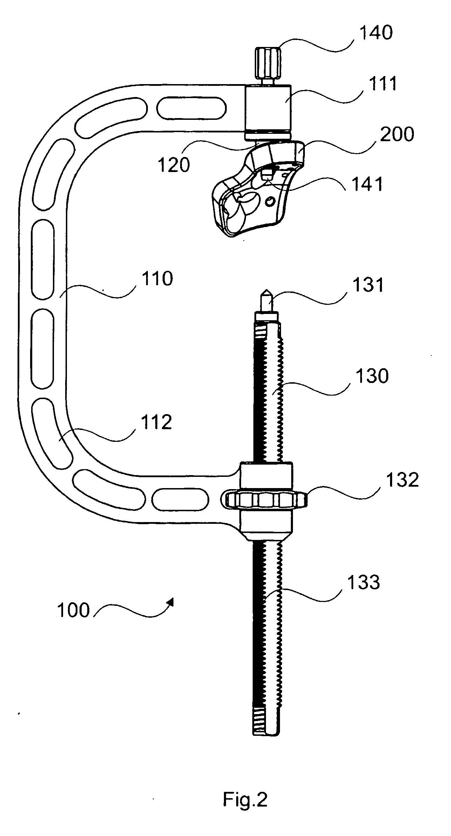

Aus Fig.1 ist ein Zielgerätes 100 mit separater Bohrbüchse 140 und Zielplatte 200 ersichtlich. Das Zielgerät 100 besteht aus einem U-förmigen Bügel 110 mit diversen Durchbrechungen 112. Am untern Ende des Bügels 110 ist eine mittels einer Mutter 132 verstellbare Schraubspindel 130 gelagert. Am einen Ende der Schraubspindel 130 ist ein rotationsbeweglicher Stift 131 angeordnet. An der Schraubspindel 130 ist eine Skala 133 angebracht. Am obern Ende des Bügels 110 ist eine Zylinderführung 111 angebracht, welche ihrerseits eine Adaptionsbüchse 120 rotationsbeweglich lagert. Zylinderführung 111 und Adaptionsbüchse 120 weisen einen Innendurchmesser auf, der geeignet ist, einerseits eine Knochenschraube (nicht dargestellt) zu führen, beziehungsweise andererseits eine Bohrbüchse 140 zu lagern. Diese Bohrbüchse 140 ist an ihrem Ende mit einem Bohrbüchsengewinde 141 ausgestattet, welches bei der Anwendung eines Implantats (nicht dargestellt) mit diesem im Eingriff steht. Die Adaptionsbüchse 120 ist mit einem Schnappelemet ausgestattet, welches sein Gegenstück in einer Bohrung 220 einer Zielplatte 200 findet. Die Zielplatte 200 ist also dafür vorgesehen, mit dem Zielgerät 100 vorübergehend eine Steckverbindung einzugehen, wie es in Fig.2 dargestellt ist.

Fig.2 zeigt das Zielgerät 100 mit montierter Bohrbüchse 140 und Zielplatte 200. Die Zielplatte 200 steht in einer Steckverbindung mit der in Fig. 1 beschriebenen Adaptionsbüchse 120. Die Bohrbüchse 140 ist in die Öffnung in der Zylinderführung 111 eingeführt und kommt, durch die Adaptionsbüchse und die Zielplatte 200 hindurch, mit ihrem Bohrbüchsengewinde 141 zum Vorschein.

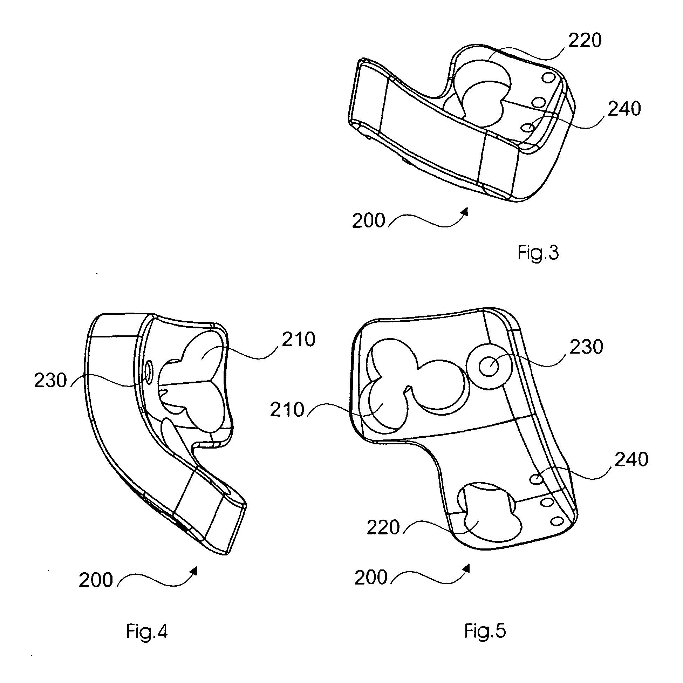

Fig.3 bis 5 zeigen die Zielplatte 200 in drei unterschiedlichen Ansichten. Diese weist eine Bohrung 220 für die Adaptionsbüchse (nicht dargestellt) auf, welche hierdurch eine Steckverbindung mit der Zielplatte 200 eingehen kann. Des weiteren sind drei Bohrungen 210 für anterior-posteriore Knochenschrauben ausgebildet, eine Bohrung 230 für eine Zielplattenschraube sowie drei Bohrungen 240 für Kirschnerdrähte.

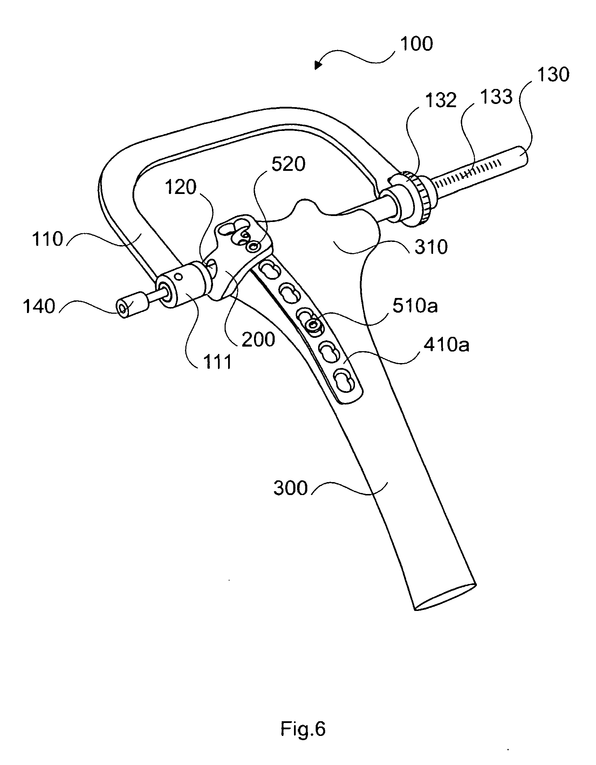

Fig.6 zeigt eine schematische Darstellung des Zielgerätes 100 mit montierter Bohrbüchse 140, Zielplatte 200 und damit verschraubtem lateralem Implantat 410a, wie es auf dem artikulären Block 310 des Humerus 300 angebracht wird. Das Instrumentarium ist also, wie in Fig. 2 beschrieben montiert, wobei zusätzlich das laterale Implantat 410a einerseits mittels einer Zielplattenschraube 520 durch die in Fig.3 bis 5 dargestellte Bohrung 230 für die Zielplattenschraube (nicht sichtbar) hindurch mit der Zielplatte 200 verschraubt ist. Andererseits steht ein nicht sichtbares Gewinde des lateralen Implantats 410a mit der Bohrbüchse 140, insbesondere mit deren Bohrbüchsengewinde 141, welches durch die Adaptionsbüchse 120 hindurchtritt, im Eingriff. Das gesamte Gebilde ist durch die mittels der Mutter 132 betätigbare Schraubspindel 130 am artikulären Block 310 des Humerus 300 festgeklemmt und mittels einer proximalen Knochenschraube 510a durch das Implantat 410a hindurch im Humerus 300 verankert.

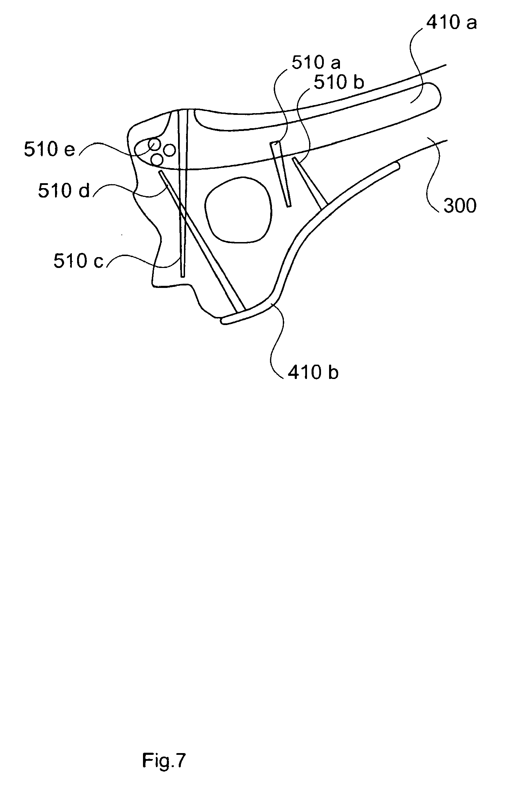

Fig.7 ist eine schematische Darstellung des Humerus mit Implantaten und Knochenschrauben in Frontalebene. Das laterale Implantat 410a ist mittels proximaler 510a, distaler 510c und anterior-posterioren 510e Knochenschrauben fixiert. Das mediale Implantat 410b ist mittels proximaler 510b und distaler 510d Knochenschrauben fixiert. Insbesondere sind die Knochenschrauben 510c und 510d ohne Penetration ins Gelenk möglichst lang gewählt und möglichst nahe aneinander vorbeigeführt.

### Bezugszeichenliste

- 100 -: Zielgerät
- 110 -: Bügel
- 111 -: Zylinderführung
- 112 -: Durchbrechung
- 120 -: Adaptionsbüchse bzw. Kontaktelement
- 130 -: Schraubspindel
- 131 -: Stift
- 132 -: Mutter bzw. Drehgriff
- 133 -: Skala
- 140 -: Bohrbüchse
- 141 -: Bohrbüchsengewinde
- 200 -: Zielplatte
- 210 -: Bohrung für anterior-posteriore Knochenschraube
- 220 -: Bohrung für Adaptionsbüchse
- 230 -: Bohrung für Zielplattenschraube
- 240 -: Bohrung für Kirschnerdraht
- 300 -: Knochen (Humerus)
- 310 -: artikulärer Block (des Humerus)
- 410 -: Implantat
- 410a -: laterales Implantat
- 410b -: mediales Implantat
- 510 -: Knochenschraube
- 510a -: proximale Knochenschraube (lateral)
- 510b -: proximale Knochenschraube (medial)
- 510c -: distale Knochenschraube (lateral)
- 510d -: distale Knochenschraube (medial)
- 510e -: anterior-posteriore Knochenschraube
- 520 -: Zielplattenschraube

## Patentansprüche

1. Set zum Bohren eines Loches im artikulären Bereich eines Knochens (300), bestehend aus
a) einem Zielgerät (100), bestehend aus einem U-förmigen Bügels (110) mit wenigstens einem Kontaktelement (120) an einem ersten Ende des Bügel (110) und einer in Richtung oder Gegenrichtung des Kontaktelements (120) beweglichen Schraubspindel (130) mit einem Drehgriff (132) am anderen, zweiten Ende des Bügels (110), zum Festklemmen des Gerätes (100) am artikulären Bereich, sowie einer Bohrbüchse (140),
b) einem Implantat (410),
c) einer Zielplatte (200), wobei die Bohrbüchse (140) in einer zylindrischen Führung (111) an dem ersten Ende des U-förmigen Bügels (110) angeordnet ist, **dadurch gekennzeichnet, dass** das Kontaktelement (120) des Zielgeräts (100) vorübergehend eine Steckverbindung mit der Zielplatte (200) eingehen kann, und
die Bohrbüchse (140) am Implantat (410) befestigt und entfernt werden kann, sodass im Anwendungsfall die mittels der Schraubspindel (130) erzeugte Kompression des Knochens (300) nach dem Entfernen der Bohrbüchse (140) für das Einbringen einer Knochenschraube (510) in den Knochen (300) durch ein Gewinde des Implantats (410) zwischen Kontaktelement (120) und Zielplatte (200) erhalten bleibt.

2. Set nach Anspruch 1, **dadurch gekennzeichnet, dass** das Kontaktelement als rotationsbewegliche Adaptionsbüchse (120) ausgestaltet ist.

3. Set nach Anspruch 2, **dadurch gekennzeichnet, dass** die Zielplatte (200) auf dem Implantat (410) verschraubbar ist.

4. Set nach Anspruch 2 oder 3, **dadurch gekennzeichnet, dass** die Bohrbüchse (140) durch die Adaptionsbüchse (120) hindurch führbar und mit dem Implantat (410) in Eingriff bringbar ist.

5. Set nach einem der vorhergehenden Ansprüche, **dadurch gekennzeichnet, dass** auf der Schraubspindel (130) oder einem mit dieser fix verbundenen oder im Eingriff stehenden Element eine Skala (133) zur Längenermittlung einer in das Loch einzuführenden Knochenschraube (510) angebracht ist

6. Set nach einem der vorhergehenden Ansprüche, **dadurch gekennzeichnet, dass** das dem Kontaktelement (120) zugewandten Ende der Schraubspindel (130) einen rotationsbeweglichen gelagerten, vorzugsweise auswechselbaren Stift (131) aufweist.

7. Set nach einem der vorhergehenden Ansprüche, **dadurch gekennzeichnet, dass** der Drehgriff als eine auf der Schraubspindel (130) gelagerten Mutter (132) ausgebildet ist.

8. Set nach Anspruch 7, **dadurch gekennzeichnet, dass** die Mutter (132) in einer Aussparung des Bügels (110) gelagert ist.

9. Set nach einem der vorhergehenden Ansprüche, **dadurch gekennzeichnet, dass** der Bügel (110) als Gitterstruktur ausgebildet ist oder verschiedene Durchbrechungen (12) aufweist.

10. Set nach einem der vorhergehenden Ansprüche, **gekennzeichnet dadurch, dass** das Implantat (410) mittels der Bohrbüchse (140) mit der Zielplatte (200) temporär fixierbar ist und, dass das Set zum Einbringen von winkelstabilen, langen Schrauben im artikulären Bereich eines Knochens mit einem Zielgerät (100) geeignet ist.

11. Set, nach Anspruch 10, **dadurch gekennzeichnet, dass** die temporäre Fixierung der Zielplatte (200) mit dem Implantat (410) mittels der Bohrbüchse 140) über eine Gewindeverbindung erfolgt.

## Claims

1. Set for boring a hole in the particular area of a bone (300) comprising a) an aiming device (100) consisting of a U-shaped bracket (110) with at least one contact element (120) on a first end of the bracket (110) and a screw spindle (130), movable in the direction of or counter to the contact element (120), with a rotating grip (132) on the other, second end of the bracket (110), for clamping the device (100) to the articular area, as well as a boring bush (140), b) an implant (410) and c) a target plate (200) and the contact element (120) of the aiming device (100) can temporarily form a plug-type connection with the target plate (200), whereby the boring bush (140) is arranged in a cylindrical guide (111) on the first end of the U-shaped bracket (110) **characterised in that** the boring bush (140) can be attached to and removed from the implant (410) so that when in use the compression of the bone (300) brought about by the screw spindle (130) remains in place after removal of the boring bush (140) for inserting a bone screw (510) into the bone (300) by way of a thread of the implant (410) between contact element (120) and target plate (200).

2. Set in accordance with claim 1 **characterised in that** the contact element is designed as a rotating adaptation bush (120).

3. Set in accordance with claim 2 **characterised in that** the target plate (200) can be screwed onto the implant (410).

4. Set in accordance with claim 2 or 3 **characterised in that** the boring bush (140) can be passed through the adaptation bush (120) and brought into engagement with the implant (410).

5. Set in accordance with any one of the preceding claims **characterised in that** on the screw spindle (130) or an element firmly connected or in engagement therewith, a scale (133) is fitted for determining the length of a bone screw (510) to be introduced into the hole.

6. Set in accordance with any one of the preceding claims **characterised in that** the end of the screw spindle (130) facing the contact element (120) has a rotationally borne, preferably interchangeable pin (131).

7. Set in accordance with any one of the preceding claims **characterised in that** the rotating grip is in the form of a nut (132) borne on the screw spindle (130).

8. Set in accordance with claim 7 **characterised in that** the nut (132) is arranged in a recess of the bracket (110).

9. Set in accordance with any one of the preceding claims **characterised in** tat the bracket (110) is of a latticework structure or has various perforations (112).

10. Set in accordance with any ane of the preceding claims **characterised in that** the implant (410) can be temporarily fixed by means of the boring bush (140) to the target plate (200) and that the set is suitable for the insertion of angle-stable, long screws into the articular area of a bone with an aiming device (100).

11. Set in accordance with claim 10 **characterised in that** the temporary fixing of the target plate (200) to the implant (410) by means of the boring bush (140) takes place via a threaded connection.

## Revendications

1. Kit de perçage d'un trou dans la zone articulaire d'un os (300), composé a) d'un appareil cible (100) composé d'un étrier en forme de U (110) pourvu d'au moins un élément de contact (120) à une première extrémité de l'étrier (110) et d'une broche filetée (130) mobile dans la direction ou la direction opposée de l'élément de contact (120) avec une poignée rotative (132) à l'autre seconde extrémité de l'étrier (170) pour bloquer l'appareil (100) au niveau de la zone articulaire ainsi que d'un canon de perçage (140), b) d'un implant (410), le canon de perçage (140) étant disposé dans un guide cylindrique (111) à la première extrémité de l'étrier en forme de U (110), c) d'une plaque cible (200), **caractérisé en ce que** l'élément de contact (120) de l'appareil cible (100) peut établir provisoirement un raccord fiché avec la plaque cible (200) et que le canon de perçage (140) peut être fixé à l'implant (410) et en être enlevé, de sorte que, dans le cas d'application, la compression de l'os (300) générée au moyen de la broche filetée (130) persiste après l'enlèvement du canon de perçage (140) pour l'insertion d'une vis à os (510) dans l'os (300) à travers un filetage de l'implant (410) entre l'élément de contact (120) et la plaque cible (200).

2. Kit selon la revendication 1, **caractérisé en ce que** l'élément de contact est réalisé sous forme d'un manchon adaptateur mobile en rotation (120).

3. Kit selon la revendication 2, **caractérisé en ce que** la plaque cible (200) peut être vissée sur l'implant (410).

4. Kit selon la revendication 2 ou 3, **caractérisé en ce que** le canon de perçage (140) peut être passé à travers le manchon adaptateur (120) et être mis en prise avec l'implant (410).

5. Kit selon une des revendications précédentes, **caractérisé en ce que**, sur la broche filetée (130) ou sur un élément relié fixement à celle-ci ou en prise avec, est installée une écuelle (133) permettant de déterminer la longueur d'une vis à os (510) à introduire dans le trou.

6. Kit selon une des revendications précédentes, **caractérisé en ce que** l'extrémité tournée vers l'élément de contact (120) de la broche filetée (130) présente une tige (131) s'appuyant de manière mobile en rotation et de préférence échangeable.

7. Kit selon une des revendications précédentes, **caractérisé en ce que** la peignée rotative est réalisée sous forme d'un écrou (132) s'appuyant sur la broche filetée (130).

8. Kit selon la revendication 7, **caractérisé en ce que** l'écrou (132) s'appuie dans un évidement de l'étrier (110).

9. Kit selon une des revendication précédentes, **caractérisé en ce que** l'étrier (110) est réalisé sous forme d'une structure grillagée ou présente différentes percées (112).

10. Kilt selon une des revendications précédentes, **caractérisé en ce que** l'implant (410) peut être fixé temporairement au moyen du canon de perçage (140) à la plaque cible (200) et que ce kit est adapté pour l'insertion de vis langues à angle stable dans la zone articulaire d'un os dotée d'un appareil cible (100).

11. Kit selon la revendication 10, **caractérisé en ce que** la fixation temporaire de la plaque cible (200) à l'implant (410) se fait au moyen du canon de perçage (140) via un raccord fileté.
